# EUROPÄISCHE PATENTSCHRIFT

(11) **EP 2 114 861 B1**
(45) Veröffentlichungstag und Bekanntmachung des Hinweises auf die Patenterteilung: **05.09.2012**
(21) Anmeldenummer: 08709228.4
(22) Anmeldetag: 27.02.2008
(51) Int. Cl.: C07C 209/48, C07C 211/14, C07C 255/25

(54) **VERFAHREN ZUR HERSTELLUNG VON TRIETHYLENTETRAAMIN**
METHOD FOR PRODUCING TRIETHYLENETETRAMINE
PROCÉDÉ DE FABRICATION DE TRIÉTHYLÈNE-TÉTRAAMINE

(30) Priorität: 01.03.2007 EP 07103286
(43) Veröffentlichungstag der Anmeldung: 11.11.2009
(73) Patentinhaber: BASF SE, 67056 Ludwigshafen (DE)
(72) Erfinder: DAHMEN, Kirsten, 67251 Freinsheim (DE); OFTRING, Alfred, 67098 Bad Dürkheim (DE); HUGO, Randolf, 67246 Dirmstein (DE); BAUMANN, Katrin, 68529 Mannheim (DE); HAHN, Thilo, 67292 Krichheimbolanden (DE); MELDER, Johann-Peter, 67459 Böhl-Iggelheim (DE)
(86) Internationale Anmeldenummer: PCT/EP2008/052338
(87) Internationale Veröffentlichungsnummer: WO 2008/104553

(56) Entgegenhaltungen:
- US-A1- 2002 058 842
- US-A1- 2006 041 170
- Y. LI ET AL: "The syntheses of cyclic spermine alkaloids: analogues of Buchnerine and Budmunchiamine C" HELVETICA CHIMICA ACTA., Bd. 86, 2003, Seiten 310-323, XP002479383 CHVERLAG HELVETICA CHIMICA ACTA. BASEL.
- J. A. SPICER ET AL: "Synthesis and evaluation of unsymmetrical bis(arylcarboxamides)designed as topoisomerase-targeted anticancer agents" BIOORGANIC & MEDICINAL CHEMISTRY., Bd. 10, 2002, Seiten 19-29, XP002479382 GBELSEVIER SCIENCE LTD.
- S. A. GAMAGE ET AL: "Dicationic bis(9-methylphenazine-1-carboxamides):Rela tionships between biological activity and linker chain structure for a series of potnet topoisomerase targeted anticancer drugs" JOURNAL OF MEDICINAL CHEMISTRY., Bd. 44, 2001, Seiten 1407-1415, XP002479251 USAMERICAN CHEMICAL SOCIETY. WASHINGTON.

## Beschreibung

Die Erfindung betrifft ein Verfahren zur Herstellung von Triethylentetraamin (TETA) durch Hydrierung von Ethylendiamindiacetonitril (EDDN) an einem Katalysator. Gegebenenfalls kann EDDN auch als Bestandteil eines Aminonitrilgemisches vorliegen, das zusätzlich Ethylendiaminmonoacetonitril (EDMN) enthält.

Es ist generell bekannt, dass aliphatische Nitrile, die gegebenenfalls noch mit weiteren funktionellen Gruppen substituiert sind, in Gegenwart von Katalysatoren zu den entsprechenden Aminen hydriert werden können. Wie nachfolgend aufgezeigt, sind solche Hydrierverfahren auch für diverse Aminonitrile zur Herstellung von einigen Aminen bekannt. Bis jetzt ist jedoch nirgendwo beschrieben worden, dass auch TETA ausgehend von dem Aminonitril EDDN oder gegebenenfalls von einem Aminonitrilgemisch enthaltend EDDN und EDMN durch direkte Hydrierung des Aminonitrils hergestellt werden kann. Die bisher bekannten Verfahren zur Herstellung von TETA sind jedoch, wie nachfolgend aufgeführt, mit Nachteilen verbunden.

Im Stand der Technik sind zahlreiche Verfahren zur Hydrierung der α-Aminonitrile Aminoacetonitril (AAN) und Iminodiacetonitril (IDAN) öder von β-Aminonitrilen beschrieben. So ist es bekannt, dass die Hydrierung von β-Aminonitrilen in aller Regel problemlos verläuft, während die Hydrierung von α-Aminonitrilen mit dem Auftreten von zahlreichen Nachteilen verbunden ist, wie der Hydrogenolyse der C-CN-Bindung oder der R₂N-C-Bindung. "Handbook of Heterogeneous Catalytic Hydrogenation for Organic Synthesis, S. 213 - 215" zeigt die Problematik der Hydrierung von α-Aminonitrilen anhand von α-Alkylaminonitrilen oder cyclischen α-Aminonitrilen im Vergleich zu β-Aminonitrilen auf. Die bekannten Stabilitätsprobleme der α-Aminonitrile sind vermutlich der Hauptgrund dafür, warum bis heute nur die Hydrierung der α-Aminonitrile AAN oder IDAN zu EDA (Ethylendiamin) bzw. DETA (Diethylentriamin) genauer beschrieben wird. Großtechnisch werden EDA oder DETA jedoch durch die nachfolgend beschriebenen EDC- oder MEOA-Verfahren hergestellt. Für höhere α-Aminonitrile ist eine entsprechende Hydrierung jedoch nicht bekannt.

In DE-A 3 003 729 wird ein Verfahren zum Hydrieren von aliphatischen Nitrilen, Alkylenoxynitrilen und Alkylenaminonitrilen zu primären Aminen in Anwesenheit eines Lösungsmittelsystems an einem Kobalt- oder Rutheniumkatalysator beschrieben. Das verwendete Lösungsmittelsystem enthält neben Wasser und Ammoniak einen Ether oder Polyether. Die als Edukte einsetzbare Alkylenaminonitrile oder Alkylenoxynitrile sind jeweils über komplexe allgemeine Formeln definiert. Unter anderem sind als konkrete Verbindungen oder Beispiele, die zum entsprechenden Diamin hydriert werden können, Ethylendiamindipropionitril (EDDPN; auch als N,N'-Bis(cyanoethyl)-ethylendiamin bezeichnet) oder 3,3'-(Ethylendioxy)-dipropionitril aufgeführt. DE-A 3 003 729 offenbart hingegen keinen Hinweis auf die Verwendung von Einzelverbindungen von EDA-Derivaten mit Cyanomethylsubstituenten, wie EDDN oder EDMN. Letztgenanntes fällt zudem nicht unter die allgemeine Definition von Alkylenaminonitrilen gemäß diesem Dokument.

EP-A 0 382 508 beschreibt ein Verfahren zur chargenweisen Herstellung von nicht-cyclischen, aliphatischen Polyaminen durch Hydrierung von nicht-cyclischen, aliphatischen Polynitrilen in flüssiger Phase an Raney-Kobalt-Katalysatoren, vorzugsweise in Gegenwart von wasserfreiem Ammoniak. Hierbei wird eine Polynitrillösung einer Reaktionszone zugeführt, die den Raney-Kobalt-Katalysator in einer im wesentlichen sauerstofffreien Atmosphäre enthält. Während des gesamten Reaktionszeitraums wird die Polynitrillösung mit einer Rate zugeführt, die nicht größer ist als die maximale Rate, mit der das Polynitril mit dem Wasserstoff in der Reaktionszone reagiert. Mit diesem Verfahren können Polyamine aus Polynitrilen, wie Iminodiacetonitril (IDAN), Nitrilotriacetonitril, Ethylendiamintetraacetonitril (EDTN) oder weitere nicht näher spezifizierte Verbindungen mit 2 oder mehr Cyanogruppen hergestellt werden.

EP-A 212 986 betrifft ein weiteres Verfahren, bei dem die gleichen aliphatischen Polynitrile wie in EP-A 0 382 508 in Gegenwart eines im Eduktstrom enthaltenen, flüssigen primären oder sekundären Amines an einem granularen Raney-Kobalt-Katalysators zu den entsprechenden Polyaminen hydriert werden können. Unter anderem ist als zwingend vorhandene Aminokomponente Ethylendiamin (EDA) neben zahlreichen weiteren primären oder sekundären Aminen aufgeführt.

EP-A 1 209 146 betrifft ein weiteres Verfahren zur kontinuierlichen Hydrierung von Nitrilen zu primären Aminen, wobei die jeweiligen Nitrile in flüssiger Phase an einem suspendierten, aktivierten Raney-Katalysator auf Basis einer Legierung aus Aluminium eingesetzt werden und die Reaktion in Abwesenheit von Ammoniak und basischen Alkali- oder Erdalkaliverbindungen durchgeführt wird. Als Nitrile können neben vielen anderen auch IDAN, EDTN, EDDPN oder Ethylendiaminmonopropionitril (EDMPN) zu den entsprechenden Ethylenaminen umgesetzt werden.

EP-B 0 913 388 betrifft ein Verfahren zum katalytischen Hydrieren von Nitrilen, das den Kontakt des Nitrils mit Wasserstoff in Gegenwart eines Kobaltschwammkatalysators bei Bedingungen für die Durchführung der Umwandlung der Nitrilgruppe in das primäre Amin umfasst. Der Kobaltschwammkatalysator ist zuvor mit einer katalytischen Menge von Lithiumhydroxid behandelt worden und das Verfahren wird in Gegenwart von Wasser durchgeführt. Als Nitrile eignen sich aliphatische Nitrile mit 1 bis 30 Kohlenwasserstoffatomen, unter anderem auch β-Aminonitrile wie Dimethylaminopropionitril. Ein weiteres Verfahren zur Herstellung von Polyaminen aus den entsprechenden Polynitrilen wird in DE-A 27 55 687 offenbart. In diesem Verfahren wird die Hydrierung an einem Hydrierungskatalysator in Tablettenform in Gegenwart eines den Zerfall des Katalysators inhibierenden Stabilisators durchgeführt. Als Polynitril kann unter anderem Ethylendiamindipropionitril (EDDPN) verwendet werden. Als Stabilisator eignet sich unter anderem EDA.

US-A 2006/0041170 betrifft ein Verfahren zur Herstellung von TETA, insbesondere von TETA-Salzen, und deren Verwendung als Arzneimittel. In diesem mehrstufigen Verfahren wird zunächst EDDN hergestellt. Anschließend wird EDDN mit Benzaldehyd unter Ausbildung eines (cyclischen) Imidazolidin-Derivates umgesetzt. Diese cyclische Verbindung, die zwei Cyano-Gruppen aufweist wird reduziert, beispielsweise durch Umsetzung mit Wasserstoff, wobei die entsprechende cyclische Diamino-Verbindung erhalten wird. Diese Diamino-Verbindung wird wiederum in Gegenwart einer Säure hydrolisiert unter Erhalt des entsprechenden TETA-Salzes. In einer alternativen Ausführungsform wird die cyclische Diamin-Verbindung ebenfalls mit Benzaldehyd umgesetzt unter Ausbildung der entsprechenden Diimin-Verbindung, die anschließend wiederum in Gegenwart einer Säure unter Erhalt des entsprechenden TETA-Salzes hydrolisiert wird. Als weitere Verfahrensalternative wird in diesem Dokument die Umsetzung von EDDN mit Boc-Schutzgruppen (Tertiär-Butoxy-Carbonyl-Gruppen) beschrieben. Das dabei erhaltene, zweifach mit Boc-Schutzgruppen geschützte EDDN-Derivat wird anschließend unter Erhalt des entsprechenden geschützten TETA-Derivates hydriert. Die Entfernung der Boc-Schutzgruppen erfolgt durch saure Hydrolyse unter Erhalt des entsprechenden TETA-Salzes. Nachteilig an diesem in US-A 2006/0041170 beschriebenen Verfahren ist insbesondere, dass es sich um ein mehrstufiges Hydrierverfahren handelt, bei dem das eingesetzte Edukt EDDN zuerst chemisch derivatisiert werden muss, um die Hydrierung durchzuführen. Weiterhin ist nachteilig, dass zunächst TETA als Salz und nicht in der freien Basenform anfällt.

Im Stand der Technik wird somit nirgendwo beschrieben, dass EDDN oder Aminonitrilgemische enthaltend EDDN und EDMN durch direkte Hydrierung des Aminonitrils zur Herstellung von TETA und gegebenenfalls weiterer Ehtylenamine eingesetzt werden können. Andere (großtechnische) Verfahren zur Herstellung von TETA sind jedoch bekannt.

EP-A 222 934 betrifft ein Verfahren zur Herstellung von höheren Alkylenpolyaminen durch Umsetzung eines vicinalen Dihaloalkans mit einem Überschuss an Ammoniak in wässrige Phase unter Zugabe einer starken Base, wobei ein Imin-Zwischenprodukt gebildet wird, das anschließend mit einem Alkylenpolyamin unter Ausbildung des höheren Alkylenpolyamins umgesetzt wird. Als vicinales Dihaloalkan eignet sich insbesondere Ethylendichlorid (EDC oder 1,2-Dichlorethan). Als Alkylenpolyamine werden insbesondere Ethylendiamin oder höhere Ethylenamine wie DETA, aber auch TETA und Tetraethylenpentaamin (TEPA) eingesetzt. Bei diesen Verfahren (EDC-Verfahren) fällt ein Gemisch verschiedener Ethylenamine (lineare Ethylenamine wie EDA, DETA, TE-TA, TEPA oder höhere Ethylenamine sowie cyclische Derivate wie Piperazin (Pip) oder Aminoethyl-Piperazin (AEPip)) an. Je nachdem, welches Ethylenamin zu den Edukten EDC und NH₃ zugegeben wird, enthält das Reaktionsgemisch einen entsprechenden Anteil an höheren Ethylenaminen. Wenn beispielsweise gezielt TEPA hergestellt werden soll, wird den Edukten EDC und NH₃ das Ethylenamin TETA zugegeben. Auf diese Weise enthält das Produkt (Ethylenamingemisch) einen höheren Anteil an TEPA, jedoch auch die vorgenannten weiteren linearen sowie cyclischen Ethylenamine. Nachteilig an diesem Verfahren ist insbesondere, dass das Verfahren mit einer geringen Selektivität abläuft (bezüglich die Komponenten des erhaltenen Ethylenamingemisches) und dass zuerst ein spezielles Ethylenamin hergestellt werden muss (beispielsweise DETA), welches anschließend in das Verfahren eingebracht wird, um das nächst höhere Ethylenamin (beispielsweise TETA) gezielt herzustellen bzw. die Ausbeute zu erhöhen. Dieses Verfahren stellt außerdem aufgrund der eingesetzten Edukte (Halogenalkane) sowie der anfallenden Salzsäure ein Korrosionsproblem sowie aufgrund der anfallenden Salze ein Umweltproblem dar.

US-A 3,462,493 betrifft ein Verfahren zur Herstellung von TETA, wobei ein mindestens fünffach molarer Überschuss an EDA mit Ethylendichlorid oder Ethylendibromid umgesetzt wird. Als Nebenprodukte treten hierbei insbesondere Pip oder Piperazinoethylethylendiamin auf.

DE-T 689 11 508 beschreibt ein alternatives Verfahren zur Herstellung von linear verlängerter Polyalkylenpolyamine wie TETA. In diesem Verfahren wird ein bifunktioneller aliphatischer Alkohol mit einem Aminreaktanden in Anwesenheit eines Wolframhaltigen Katalysators umgesetzt. Als bifunktionaler aliphatischer Alkohol eignet sich insbesondere Monoethanolamin (MEOA), als Aminreaktanden können beispielsweise EDA oder DETA eingesetzt werden. Durch dieses Verfahren werden prinzipiell Gemische aus linear verlängerten Polyalkylenpolyaminen (also Ethylenamingemische) erhalten. In diesen Ethylenamingemischen ist an Ethylenaminen DETA, TETA, TEPA, Pip, AEPip oder Piperazinderivate höherer Ethylenamine enthalten, wobei der Anteil der jeweiligen Komponente je nach eingesetzten Aminreaktanden variiert. Sofern als Aminreaktand DETA verwendet wird, wird ein Ethylenamingemisch mit hohem Anteil an TETA und TEPA erhalten. An diesem Verfahren ist nachteilig, dass das Verfahren mit einer geringen Selektivität abläuft (bezüglich die Komponenten des erhaltenen Ethylenamingemisches) und dass zuerst ein zusätzliches Ethylenamin synthetisiert werden muss, dass mit dem bifunktionalen aliphatischen Alkohol (beispielsweise MEOA) umgesetzt wird. Hierbei entstehen in größerer Menge Nebenprodukte wie Aminoethylethanolamin (AEEA) oder höhere Hydroxy-haltige Ethylenamine, die von geringerem wirtschaftlichem Interesse sind. Die größere Menge an anfallenden Nebenprodukten ist darin begründet, dass MEOA bzw. die höheren Ethanolamine (z.B. AEEA) anstelle mit dem eingesetzten Amin mit sich selbst reagieren können. Aufgrund der (statistisch) vielen Reaktionsmöglichkeiten ist die Selektivität zum linearen TETA wegen der Koppelprodukte recht gering und nicht steuerbar. Die Synthese ist nur bei Teilumsatz möglich.

Einen Überblick über die Herstellung von Ethylenaminen liefert der SRI-Report "CEH Product Review Ethyleneamines"; SRI. International, 2003; S. 53-54, in dem entsprechend der vorstehend beschriebenen Verfahren (mit den Edukten EDC oder MEOA) insbesondere EDA oder DETA hergestellt werden. Dabei fallen höhere Ethylenamine wie TETA oder TEPA als Nebenprodukte an bzw. werden durch erneute Umsetzung der Edukte mit EDA oder DETA in höherer Ausbeute erhalten.

Aufgabe der vorliegenden Erfindung ist es demnach, ein einfaches und kostengünstiges Verfahren zur gezielten Herstellung von TETA.

Die Aufgabe wird gelöst durch ein Verfahren zur Herstellung von Triethylentetraamin (TETA), wobei Ethylendiamindiacetonitril (EDDN) in Gegenwart eines Katalysators und eines Lösungsmittels hydriert wird und der Druck 30 bis 250 bar beträgt. Sofern EDDN in einem Aminonitrilgemisch enthaltend mindestens 30 Gew.-% EDDN und mindestens 5 Gew.-% Ethylendiaminmonoacetonitrol (EDMN) vorliegt, wird neben TETA als weiteres Hauptprodukt auch DETA erhalten. Hydrieren im Rahmen der vorliegenden Erfindung bedeutet die Reaktion von EDDN oder gegebenenfalls weiterer Aminonitrile mit Wasserstoff.

Das erfindungsgemäße Verfahren hat den Vorteil, dass TETA bei hohem Umsatz und/oder Selektivität hergestellt werden können. Die erhöhte Selektivität zeigt sich insbesondere darin, dass das eingesetzte EDDN überwiegend zu TETA hydriert wird. Die dabei gebildeten Nebenprodukte sind hauptsächlich weitere lineare Ethylenamine wie DETA oder TEPA. Der Anteil an cyclischen Ethylenaminen wie AEPip ist im erfindungsgemäßen Verfahren relativ gering: Die weiteren Ethylenamine sind jedoch teilweise ebenfalls interessante Wertprodukte (hauptsächlich die linearen Ethylenamine wie DETA), deren Isolierung beispielsweise in großtechnischen Verfahren lohnenswert ist. Cyclische Ethylenamine wie AEPip sind hingegen als Wertprodukt von eher geringem Interesse. AEPip kann jedoch wieder in die Aminonitrilsynthese zurückgeführt werden und ergibt nach anschließender Hydrierung ebenfalls Wertprodukt, das auch als "technisches TETA" bezeichnet wird.

In vorteilhafter Weise wird EDDN und gegebenenfalls EDMN vollständig oder nahezu vollständig umgesetzt. Dies ist insbesondere bei großtechnischen Verfahren von Bedeutung, da nicht umgesetztes Edukt in der Regel in den Produktionskreislauf zurückgeführt wird bzw. entsorgt werden muss. Verfahren, bei denen größere Mengen an EDDN und/oder EDMN nicht umgesetzt werden, sind aufgrund der hohen Instabilität des EDDN bzw. EDMN von besonderem Nachteil. Einerseits neigen sowohl EDDN als auch EDMN sich bei höheren Temperaturen zu zersetzen, so dass die Zersetzungsprodukte nicht in den jeweiligen Kreislauf zurückgeführt werden können, andererseits kann diese Zersetzung auch mit explosionsartiger Heftigkeit verlaufen. Da im erfindungsgemäßen Verfahren die Aminonitrile vollständig umgesetzt werden können, müssen hinsichtlich der Rückführung in den Produktionszyklus keine Anstrengungen unternommen werden.

Ein weiterer Vorteil des erfindungsgemäßen Verfahrens ist, dass im Gegensatz zum EDC-Verfahren auf den Einsatz von chlorierten Kohlenwasserstoffen als Edukt verzichtet werden kann. Zudem fällt keine Salzsäure bzw. deren Salze als weiteres Reaktionsprodukt an. Die Entsorgung der vorgenannten Stoffe ist insbesondere bei großtechnischen Verfahren ein (Umwelt-)Problem. Vorteilhaft im Vergleich zum MEOA-Verfahren ist, dass aufgrund der unterschiedlichen Edukte die Bildung von AEEA sowie weiterer Verbindungen mit Hydroxy-Funktion keine Rolle spielt.
Sofern im erfindungsgemäßen Verfahren ein Aminonitrilgemisch hydriert wird, ist es als Vorteil anzusehen, dass, je nach Bedürfnissen des Marktes, ein höherer oder niedriger Anteil an TETA oder DETA hergestellt werden kann. Dies ist darin begründet, dass sich prinzipiell das Verhältnis der Edukte EDMN zu EDDN im Produkt hinsichtlich DE-TA zu TETA wieder findet. So können im erfindungsgemäßen Verfahren spezielle Aminonitrilgemisch-Zusammensetzungen gezielt eingesetzt werden, um die im Markt gewünschten Mengenverhältnisse zu bedienen. Das erfindungsgemäße Verfahren liefert bei hoher Selektivität ein Ethylenamingemisch, das mindestens 30 % TETA, neben mindestens 5 % DETA sowie gegebenenfalls weitere Ethylenamine wie z.B. PiperazinDerivate als Wertprodukte erhält.

Im erfindungsgemäßen Verfahren wird in einer Ausführungsform EDDN als (Haupt-)Edukt verwendet. In dieser Ausführungsform ist der Gehalt an weiteren Aminonitrilen in der Lösung, die hydriert wird, vorzugsweise auf ≤ 10 Gew.-%, insbesondere ≤ 5 Gew.-%,bezogen auf EDDN, beschränkt. In einer weiteren Ausführungsform der vorliegenden Erfindung liegt EDDN als Bestandteil eines Aminonitrilgemisches vor. Das Aminonitrilgemisch enthält neben mindestens 30 Gew.-% (Gewichtsprozent) EDDN, mindestens 5 Gew.-% Ethylendiaminomonoacetonitril (EDMN) sowie gegebenenfalls weitere Aminonitrile. EDDN ist normalerweise zu 30 bis 95 Gew.-%, bevorzugt zu 50 bis 95 Gew.-%, besonders bevorzugt zu 75 bis 90 Gew.-% im Aminonitrilgemisch enthalten. Das Aminonitrilgemisch enthält EDMN normalerweise zu 5 bis 70 Gew.-%, bevorzugt zu 5 bis 50 Gew.-%. Besonders bevorzugt enthält es EDMN zu 10 bis 25 Gew.-%. Die vorstehenden Gewichtsprozentangaben von EDDN und EDMN sowie den weiteren Aminonitrilen beziehen sich auf die Gesamtmenge der im Gemisch enthaltenen Aminonitrile. Zusätzlich vorhandenes Wasser oder sonstige Lösungsmittel sind bei diesen Mengenangaben nicht berücksichtigt.

Generell kann jede Art/Qualität von EDDN und gegebenenfalls von EDMN sowie weiteren Aminonitrilen eingesetzt werden. Bevorzugt werden die entsprechenden Aminonitrile in Form ihrer wässrigen Lösung eingesetzt. Verfahren zur Herstellung von EDDN oder EDMN sind dem Fachmann bekannt. Siehe hierzu K. Masuzawa et al., Bull. Chem. Soc. Japan, Band 41 (1968), Seiten 702-707; H. Brown et al., Helvetica Chimica Acta, Band 43 (1960), Seiten 659-666 und H. Baganz et al., Chem. Ber., 90 (1957), Seiten 2944-2949. Vorzugsweise werden EDDN und/oder EDMN durch Umsetzung von EDA und Formaldehydcyanhydrin (FACH) hergestellt. Verfahren zur Herstellung von FACH sind dem Fachmann bekannt.

Weiterhin können EDDN und/oder EDMN durch Umsetzung von EDA mit Formaldehyd und Blausäure hergestellt werden.

Prinzipiell kann EDDN vor dem Einsatz im erfindungsgemäßen Verfahren nach dem Fachmann bekannten Methoden aufgereinigt werden. Gegebenenfalls kann auch frisch hergestelltes EDDN im erfindungsgemäßen Verfahren eingesetzt werden. Im Anschluss an die EDDN-Synthese können Aufreinigungsschritte durchgeführt werden, denkbar ist eine Wasserabreicherung des hergestellten EDDN's. Sofern EDDN im erfindungsgemäßen Verfahren in einem Aminonitrilgemisch enthaltend EDDN und EDMN, können die einzelnen Komponenten dieses Aminonitrilgemisches getrennt voneinander synthetisiert werden und vor dem Einsatz im erfindungsgemäßen Verfahren in den entsprechenden Mengen zum Aminonitrilgemisch vereint werden.

In einer bevorzugten Ausführungsform der vorliegenden Erfindung werden EDDN und EDMN gemeinsam synthetisiert. Vorzugsweise werden dabei EDDN und EDMN durch Umsetzung von EDA und FACH hergestellt. Über die Konzentration von FACH kann das Verhältnis von EDDN zu EDMN im Aminonitrilgemisch eingestellt werden. Vorzugsweise ist das molare Verhältnis von EDA zu FACH 1 : 1,5 bis 1 : 2 [Mol/Mol]. Je geringer der Anteil an FACH, umso höher ist der Anteil EDMN im Aminonitrilgemisch. Vorzugsweise wird dabei ein Aminonitrilgemisch gemäß den vorstehenden Konzentrationsangaben an EDDN und EDMN hergestellt. In dem dabei erhaltenen Aminonitrilgemisch können die Konzentration an EDDN oder EDMN durch entsprechende Zugabe aufkonzentriert oder abkonzentriert werden.

In einer Ausführungsform der vorliegenden Erfindung werden vor der Hydrierung die Leichtsieder aus dem Edukt EDDN, das gegebenenfalls auch EDMN enthält, abgetrennt. Vorzugsweise wird im erfindungsgemäßen Verfahren EDDN, das gegebenenfalls EDMN enthält, eingesetzt, das weitgehend frei von Leichtsiedern ist. Sofern zur Herstellung von EDDN und gegebenenfalls EDMN FACH verwendet wird, kann die Leichtsiederabtrennung bereits vor der Umsetzung von FACH mit EDA erfolgen. Vorzugsweise werden als Leichtsieder Blausäure (HCN) abgetrennt. HCN kann dabei auch als Zersetzungsprodukt von FACH auftreten. Weiterhin kann an dieser Stelle eventuell vorhandener Ammoniak abgetrennt werden. Vorzugsweise erfolgt die Abtrennung durch Destillation, beispielsweise in Form einer Dünnschichtverdampfung wie z.B. einer Sambay-Detsillation ("Chemie Ingenieur Technik, Vol. 27, S. 257-261). Gegebenenfalls kann das Reaktionsgemisch vor der Hydrierung auch mit Stickstoff gestrippt werden.

EDDN ist bei Raumtemperatur ein Feststoff, ebenso EDMN. Folglich wird das erfindungsgemäße Verfahren in Gegenwart von einem Lösungsmittel wie einem organischen Lösungsmittel und/oder Wasser durchgeführt. Vorzugsweise wird Wasser als Lösungsmittel verwendet, gegebenenfalls können auch Gemische aus Wasser und organischen Lösungsmitteln wie Ether, insbesondere THF, oder Alkohole, insbesondere Methanol, verwendet werden. Die zusätzliche Verwendung eines organischen Lösungsmittels (inerten organischen Verbindung) neben Wasser erweist sich jedoch als vorteilhaft, da dadurch eine Stabilisierung der einzelnen Komponenten des wässrigen Aminonitrilgemisches, insbesondere in Gegenwart der resultierenden Amine, erzielt werden kann. Außerdem lässt sich durch die Verwendung von organischen Lösungsmitteln ein Spüleffekt (Reduzierung der Spülcyclen, Verminderung der Katalysatorausschleusung) an dem eingesetzten Katalysator erzielen, wodurch dessen Standzeit erhöht bzw. dessen Verbrauch erniedrigt (längere Katalysatorlebensdauer) sowie die Katalysatorbelastung verbessert werden kann. Durch die.Verwendung geeigneter Lösungsmittel kann weiterhin die Bildung von alkylierten Nebenprodukten wie AEPip vermindert werden.

Ein geeignetes Lösungsmittel, welches ein oder mehrere Komponenten umfassen kann, sollte bevorzugt folgende Eigenschaften aufweisen:
(a) das Lösungsmittel sollte stabilisierend insbesondere in Gegenwart der Produkte auf EDDN oder gegebenenfalls EDMN wirken, insbesondere deren Zersetzung bei den herrschenden Temperaturen verhindern;
(b) das Lösungsmittel sollte eine gute Wasserstofflöslichkeit zeigen;
(c) das Lösungsmittel sollte bei den Reaktionsbedingungen inert sein;
(d) das Reaktionsgemisch (EDDN, gegebenenfalls Wasser sowie Lösungsmittel) sollte unter Reaktionsbedingungen einphasig sein;
(e) die Lösemittelauswahl sollte im Hinblick auf eine bevorzugt destillative. Abtrennung des Produktes im Anschluss an die Hydrierung aus dem Produktstrom erfolgen. Wobei energie- oder apparativ-aufwändige (z.B. engsiedende Gemische oder schwierig zu trennende Azeotrope) Trennung zu vermeiden sind.
(f) das Lösungsmittel sollte gut von den Produkten abtrennbar sein, d.h. die Siedetemperatur sollte sich hinreichend von der der Produkte unterscheiden. Hierbei wird eine niedrigere Siedetemperatur als die der Produkte bevorzugt.

Mögliche Lösungsmittel (neben Wasser) sind organische Lösungsmittel, beispielsweise Amide, wie N-Methylpyrrolidon (NMP) und Dimethylformamid (DMF), aromatische und aliphatische Kohlenwasserstoffe, wie Benzol und Xylol, Alkohole wie Methanol, Ethanol, Propanol, Isopropanol, Butanol, Isobutanol, sekundäres Butanol und tertiäres Butanol, Amine, wie EDA bzw. Ethylamine, Ester, wie Essigsäuremethylester oder Essigsäureethylester und Ether, wie Diisopropylether, Diisobutylether, Glykoldimethylether, Diglykoldimethylether, Dioxan und Tetrahydrofuran (THF). Bevorzugt werden im erfindungsgemäßen Verfahren Ether verwendet, mehr bevorzugt cyclische Ether und besonders bevorzugt Tetrahydrofuran. In einer weiteren bevorzugten Ausführungsform werden Alkohole, insbesondere Methanol, als organisches Lösungsmittel verwendet.

Das Lösungsmittel wird im Gewichtsverhältnis zu dem eingesetzten Aminonitril (EDDN und gegebenenfalls EDMN) von 0,1 : 1 bis 15 : 1 eingesetzt. Die Konzentration des Aminonitrilgemisches in der Lösung, in der die Hydrierung durchgeführt wird, sollte so gewählt werden, dass eine geeignete Zuführrate bzw. Verweilzeit eingestellt werden kann. Es ist bevorzugt, das Aminonitril zu 10 bis 50 Gew.-% mit dem Lösungsmittel zu vermischen. Bezogen auf die besonders bevorzugten Lösungsmittel Methanol bzw. Tetrahydrofuran ist es beispielsweise vorteilhaft, das Aminonitril zu 20 bis 40 Gew.-% bezogen auf das Lösungsmittel einzusetzen.

Sofern Wasser vorhanden ist, liegt der Anteil an Wasser in der Lösung in einem Bereich von 0 bis 70 Gew.-%, vorzugsweise bei 10 bis 50 Gew.-%. Die Mengenangaben des Wassers beziehen sich dabei auf das Aminonitril-Wasser-Gemisch.

Gegebenenfalls können in der Lösung, in der die Hydrierung durchgeführt wird, zusätzliche Additive enthalten sein. Als Additive kommen prinzipiell Hydroxide wie Alkalimetallhydroxide, Alkoholate, Amide, Amine in Frage. Vorzugsweise eignen sich als Additive Amine, bevorzugt EDA und Ammoniak, insbesondere EDA. Weiterhin können auch saure Additive, wie zum Beispiel Silikate zusätzlich in der Lösung enthalten sein. Diese Substanzen können als Reinstoff oder gelöst in einem Lösungsmittel zugesetzt werden. Vorzugsweise wird das erfindungsgemäße Verfahren unter Zusatz von Additiven durchgeführt.

Als Katalysatoren zur Hydrierung der Nitril-Funktion zum Amin können Katalysatoren eingesetzt werden, die als aktive Spezies ein oder mehrere Elemente der 8. Nebengruppe des Periodensystems (Fe, Co, Ni, Ru, Rh, Pd, Os, Ir, Pt), bevorzugt Fe, Co, Ni, Ru oder Rh, besonders bevorzugt Co oder Ni enthalten. Darin eingeschlossen sind so genannte Skelett-Katalysatoren (auch als Raney®-Typ bezeichnet; nachfolgend auch: Raney-Katalysator), die durch Auslaugen (Aktivierung) einer Legierung aus hydrieraktivem Metall und einer weiteren Komponente (bevorzugt Al) erhalten werden. Die Katalysatoren können zusätzlich einen oder mehrere Promotoren enthalten. In einer bevorzugten Ausführungsform werden im erfindungsgemäßen Verfahren Raney-Katalysatoren eingesetzt, bevorzugt Raney-Kobalt- oder Raney-Nickel-Katalysatoren und besonders bevorzugt mit mindestens einem der Elemente Cr, Ni oder Fe dotierte Raney-Kobalt- oder mit einem der Elemente Mo, Cr oder Fe dotierte Raney-Nickel-Katalysatoren.

Die Katalysatoren können als Vollkatalysatoren oder geträgert eingesetzt werden. Als Träger kommen bevorzugt Metalloxide wie Al₂O₃, SiO₂, ZrO₂, TiO₂, Gemische von Metalloxiden oder Kohlenstoff (Aktivkohlen, Ruße, Graphit) zur Anwendung.

Die oxidischen Katalysatoren werden vor dem Einsatz außerhalb des Reaktors oder im Reaktor durch Reduktion der Metalloxide in einem Wasserstoff-enthaltendem Gasstrom bei erhöhter Temperatur aktiviert. Wenn die Katalysatoren außerhalb des Reaktors reduziert werden, kann danach eine Passivierung durch einen Sauerstoffenthaltenden Gasstrom oder die Einbettung in ein inertes Material erfolgen, um eine unkontrollierte Oxidation an Luft zu vermeiden und einen sicheren Umgang zu ermöglichen. Als inertes Material können organische Lösungsmittel wie Alkohole aber auch Wasser oder ein Amin, bevorzugt das Reaktionsprodukt, verwendet werden. Eine Ausnahme bei der Aktivierung stellen die Skelett-Katalysatoren dar, die durch Laugung mit wässriger Base, wie z. B. in EP-A 1 209 146 beschrieben, aktiviert werden können.

Je nach durchgeführtem Verfahren (Suspensionshydrierung, Wirbelschichtverfahren, Festbetthydrierung) werden die Katalysatoren als Pulver, Splitt oder Formkörper (bevorzugt Extrudate oder Tabletten) eingesetzt.

Besonders bevorzugte Festbettkatalysatoren sind die in EP-A1 742 045 offenbarten Cobalt-Vollkontakte, dotiert mit Mn, P, und Alkalimetall (Li, Na, K, Rb, Cs). Die katalytisch aktive Masse dieser Katalysatoren besteht vor der Reduktion mit Wasserstoff aus 55 bis 98 Gew.%, insbesondere 75 bis 95 Gew.%, Cobalt, 0,2 bis 15 Gew.-% Phosphor, 0,2 bis 15 Gew.-% Mangan und 0,05 bis 5 Gew.-% Alkalimetall, insbesondere Natrium, jeweils berechnet als Oxid.

Weitere geeignete Katalysatoren sind die in EP-A 963 975 offenbarten Katalysatoren, deren katalytisch aktive Masse vor der Behandlung mit Wasserstoff 22 bis 40 Gew.-% ZrO₂, 1 bis 30 Gew.-% sauerstoffhaltige Verbindungen des Kupfers, berechnet als CuO, 15 bis 50 Gew.-% sauerstoffhaltige Verbindungen des Nickels, berechnet als NiO, wobei das molare Ni : Cu-Verhältnis größer 1 ist, 15 bis 50 Gew.-% sauerstoffhaltige Verbindungen des Kobalts, berechnet als CoO, 0 bis 10 Gew.-% sauerstoffhaltige Verbindungen des Aluminiums und/oder Mangans, berechnet als Al₂0₃ bzw. MnO₂, und keine sauerstoffhaltigen Verbindungen des Molybdäns enthält, beispielsweise der in diesem Dokument offenbarte Katalysator A mit der Zusammensetzung 33 Gew.-% Zr, berechnet als ZrO₂, 28 Gew.-% Ni, berechnet als NiO, 11 Gew.-% Cu, berechnet als CuO und 28 Gew.-% Co, berechnet als CoO.

Weiterhin geeignet sind die in EP-A 696 572 offenbarten Katalysatoren, deren katalytisch aktive Masse vor der Reduktion mit Wasserstoff 20 bis 85 Gew.-% ZrO₂, 1 bis 30 Gew.-% sauerstoffhaltige Verbindungen des Kupfers, berechnet als CuO, 30 bis 70 Gew.-% sauerstoffhaltige Verbindungen des Nickels, berechnet als NiO, 0,1 bis 5 Gew.-% sauerstoffhaltige Verbindungen des Molybdäns, berechnet als MoO₃, und 0 bis 10 Gew.-% sauerstoffhaltige Verbindungen des Aluminiums und/oder Mangans, berechnet als Al₂O₃ bzw. MnO₂ enthält. Beispielsweise der in dieser Schrift konkret offenbarte Katalysator mit der Zusammensetzung 31,5 Gew.-% ZrO₂, 50 Gew.-% NiO, 17 Gew.-% CuO und 1,5 Gew.-% MoO₃. Ebenso geeignet sind die in WO-A-99/44984 beschriebenen Katalysatoren enthaltend (a) Eisen oder eine Verbindung auf der Basis von Eisen oder deren Gemische, (b) von 0,001 bis 0,3 Gew.-% bezogen auf (a) eines Promoters auf der Basis von 2, 3, 4 oder 5 Elementen ausgewählt aus der Gruppe Al, Si, Zr, Ti, V, (c) von 0 bis 0,3 Gew.-% bezogen auf (a) einer Verbindung auf der Basis eines Alkali- und/oder Erdalkalimetalls, sowie (d) von 0,001 bis 1 Gew.-% bezogen auf (a) Mangan.

Für Suspensionsverfahren werden bevorzugt Raney-Katalysatoren eingesetzt. Bei den Raney-Katalysatoren wird der aktive Katalysator als 'Metallschwamm' aus einer binären Legierung (Nickel, Eisen, Kobalt, mit Aluminium oder Silicium) durch Herauslösen eines Partners mit Säure oder Lauge hergestellt. Reste des ursprünglichen Legierungspartners wirken oft synergetisch.

Die im erfindungsgemäßen Verfahren eingesetzten Raney-Katalysatoren werden bevorzugt ausgehend von einer Legierung aus Kobalt oder Nickel, besonders bevorzugt Kobalt, und einer weiteren Legierungskomponente, die in Alkalien löslich ist, hergestellt. Bei dieser löslichen Legierungskomponente wird bevorzugt Aluminium verwendet, es können aber auch andere Komponenten wie Zink und Silicium oder Gemische aus solchen Komponenten eingesetzt werden.

Zur Aktivierung des Raney-Katalysators wird die lösliche Legierungskomponente ganz oder teilweise mit Alkali extrahiert, wofür zum Beispiel wässrige Natronlauge verwendet werden kann. Der Katalysator kann danach z. B. mit Wasser oder organischen Lösungsmittel gewaschen werden.

In dem Katalysator können einzelne oder mehrere weitere Elemente als Promotoren anwesend sein. Beispiele für Promotoren sind Metalle der Nebengruppen IB, VIB und/oder VIII des Periodensystems, wie Chrom, Eisen, Molybdän, Nickel, Kupfer usw.

Die Aktivierung der Katalysatoren durch Auslaugen der löslichen Komponente (typischerweise Aluminium) kann entweder im Reaktor selbst oder vor Einfüllen in den Reaktor erfolgen. Die voraktivierten Katalysatoren sind luftempfindlich und pyrophor und werden deshalb in der Regel unter einem Medium wie z. B. Wasser, einem organischen Lösungsmittel oder einem Stoff, der bei der erfindungsgemäßen Reaktion zugegen ist (Lösungsmittel, Edukt, Produkt) aufbewahrt und gehandhabt oder in eine organische Verbindung, die bei Raumtemperatur fest ist, eingebettet.

In einer bevorzugten Ausführungsform wird erfindungsgemäß ein Raney-Kobalt-Skelett-Katalysator eingesetzt, der aus einer Co/Al-Legierung durch Laugung mit wässriger Alkalimetallhydroxid-Lösung, z.B. Natronlauge, und nachfolgender Waschung mit Wasser erhalten wurde, und bevorzugt als Promotoren mindestens eines der Elemente Fe, Ni oder Cr enthält.

Solche Katalysatoren enthalten typischerweise neben Kobalt noch 1-30 Gew.-% Al, besonders 2-12 Gew.-% Al, ganz besonders 3 - 6 Gew.-% Al, 0 - 10 Gew.-% Cr, besonders 0,1 - 7 Gew.-% Cr, ganz besonders 0,5 - 5 Gew.-% Cr, insbesondere 1,5 - 3,5 Gew.-% Cr, 0 - 10 Gew.-% Fe, besonders 0,1 - 3 Gew.-% Fe, ganz besonders 0,2 - 1 Gew.-% Fe, und/oder 0 - 10 Gew.-% Ni, besonders 0,1 - 7 Gew.-% Ni, ganz besonders 0,5 - 5 Gew.-% Ni, insbesondere 1 - 4 Gew.-% Ni, wobei die Gewichtsangaben jeweils auf das Katalysatorgesamtgewicht bezogen sind.

Als Katalysator im erfindungsgemäßen Verfahren kann zum Beispiel vorteilhaft ein Kobalt-Skelett-Katalysator "Raney 2724" der Firma W. R. Grace & Co. eingesetzt werden. Dieser Katalysator weist folgende Zusammensetzung auf:
Al: 2-6 Gew.-%, Co: ≥ 86 Gew.-%, Fe: 0-1 Gew.-%, Ni: 1-4 Gew.-%, Cr: 1,5 - 3,5 Gew.-%.

Ebenfalls kann erfindungsgemäß ein Nickel-Skelett-Katalysator eingesetzt werden, der aus einer Ni/AI-Legierung durch Laugung mit wässriger Alkalimetallhydroxid-Lösung, z.B. Natronlauge, und nachfolgender Waschung mit Wasser erhalten wurde und bevorzugt als Promotoren mindestens eines der Elemente Fe, Cr enthält.

Solche Katalysatoren enthalten typischerweise neben Nickel noch
1 - 30 Gew.-% Al, besonders 2 - 20 Gew.-% Al, ganz besonders 5 - 14 Gew.-% Al,
0 - 10 Gew.-% Cr, besonders 0,1 - 7 Gew.-% Cr, ganz besonders 1 - 4 Gew.-% Cr, und/oder
0 - 10 Gew.-% Fe, besonders 0,1 - 7 Gew.-% Fe, ganz besonders 1 - 4 Gew.-% Fe, wobei die Gewichtsangaben jeweils auf das Katalysatorgesamtgewicht bezogen sind.

Als Katalysator im erfindungsgemäßen Verfahren kann zum Beispiel vorteilhaft ein Nickel-Skelett-Katalysator A 4000 der Firma Johnson Matthey eingesetzt werden.
Dieser Katalysator weist folgende Zusammensetzung auf

Al: ≤14 Gew.-%, Ni: ≥ 80 Gew.-%, Fe: 1-4 Gew.-%, Cr: 1-4 Gew.-%.

Die Katalysatoren können gegebenenfalls bei nachlassender Aktivität und/oder Selektivität mit den dem Fachmann bekannten Methoden, wie zum Beispiel in WO 99/33561 und den darin zitierten Schriften veröffentlicht, regeneriert werden.

Die Regenerierung des Katalysators kann im eigentlichen Reaktor (in situ) oder am ausgebauten Katalysator (ex situ) durchgeführt werden. Bei Festbettverfahren wird bevorzugt in situ regeneriert, bei Suspensionsverfahren wird bevorzugt ein Teil des Katalysators kontinuierlich oder diskontinuierliche entnommen, ex situ regeneriert und zurückgeführt.

Die Temperaturen, bei denen das erfindungsgemäße Verfahren durchgeführt wird, liegen in einem Bereich von 40 bis 150°C, bevorzugt von 70 bis 140°C, insbesondere bel 80 bis 130°C.

Der bei der Hydrierung herrschende Druck liegt bei 30 bis 250 bar, besonders bevorzugt bei 40 bis 160 bar.

In einer bevorzugten Ausführungsform wird EDDN beziehungsweise das Aminonitrilgemisch enthaltend EDDN mit einer Rate der Hydrierung zugeführt, die nicht größer ist als die Rate, mit der EDDN und gegebenenfalls die übrigen Komponenten des Aminonitrilgemisches mit Wasserstoff bei der Hydrierung reagiert.

Die Zuführrate ist bevorzugt so einzustellen, dass quasi Vollumsatz erreicht wird. Dieses wird durch Temperatur, Druck, Art des Gemisches, Menge und Art des Katalysators, des Reaktionsmediums, Durchmischungsgüte des Reaktorinhalts, Verweilzeit etc. beeinflusst.

Im erfindungsgemäßen Verfahren werden ein (oder mehrere) Lösungsmittel verwendet, wobei das Lösungsmittel zunächst vollständig mit EDDN oder dem Aminonitrilgemisch vermischt werden. Die erhaltene Lösung, die gegebenenfalls auch Additive enthalten kann, wird anschließend in das den Katalysator enthaltende Reaktionsgefäß zugeführt. Alternativ kann eine Teilmenge des Lösungsmittels auch separat von der Lösung, die EDDN, das Lösungsmittel und gegebenenfalls das Additiv enthält, in das Reaktionsgefäß zugegeben werden. Gegebenenfalls wird das Additiv getrennt, oder aber in der Teilmenge des Lösemittels gelöst, zudosiert.

Gegebenenfalls wird EDDN als wässrige Lösung zugefahren und ein organisches Lösemittel wird separat mit oder ohne Additiv zugefahren.

Gegebenenfalls kann, beispielsweise bei Semibatch-Verfahren, ein Teil des Lösungsmittels zusammen mit dem Katalysator im Reaktionsgefäß vorgelegt werden, worauf die Lösung zudosiert wird. Normalerweise erfolgt die Zuführung des in der Lösung enthaltenen EDDNs sowie der gegebenenfalls enthaltenen weiteren Aminonitrile wie EDMN mit einer Rate, die nicht größer ist als die Rate, mit der EDDN mit Wasserstoff bei der Hydrierung reagiert.

Das erfindungsgemäße Verfahren zur Herstellung von TETA durch Hydrierung von EDDN kann in üblichen für die Katalyse geeigneten Reaktionsgefäßen in einer Festbett-, Wirbelschicht-, Suspensionsfahrweise kontinuierlich, semikontinuierlich oder diskontinuierlich durchgeführt werden. Zur Durchführung der Hydrierung eignen sich Reaktionsgefäße, in denen eine Kontaktierung des Aminonitrils und des Katalysators mit dem gasförmigen Wasserstoff unter Druck möglich ist.

Die Hydrierung in Suspensionsfahrweise kann in einem Rührreaktor, Strahlschlaufenreaktor, Strahldüsenreaktor, Blasensäulenreaktor, bzw. in einer Kaskade derartiger gleicher oder verschiedener Reaktoren durchgeführt werden. Für die Hydrierung an einem Festbettkatalysator sind Rohrreaktoren aber auch Rohrbündelreaktoren denkbar.

Im Fall eines Festbettkatalysators wird in Sumpf- oder Rieselfahrweise mit dem Aminonitril beaufschlagt. Bevorzugt wird allerdings die Suspensionsfahrweise in semikontinuierlicher und bevorzugt in kontinuierlicher Fahrweise eingesetzt.

Die Hydrierung der Nitrilgruppen findet unter Freisetzung von Wärme statt, die in der Regel abgeführt werden muss. Die Wärmeabfuhr kann durch eingebaute Wärmeübertragerflächen, Kühlmantel oder außenliegende Wärmeübertrager in einem Umlaufkreis um den Reaktor erfolgen. Der Hydrierreaktor bzw. eine Hydrierreaktorkaskade kann in geradem Durchgang gefahren werden. Alternativ ist auch eine Kreislauffahrweise möglich, bei der ein Teil des Reaktoraustrages an den Reaktoreingang zurückgeführt wird, bevorzugt ohne vorherige Aufarbeitung des Kreislaufstromes. Damit lässt sich eine optimale Verdünnung der Reaktionslösung erreichen. Insbesondere kann der Kreislaufstrom mittels eines externen Wärmeübertragers auf einfache und kostengünstige Weise gekühlt und somit die Reaktionswärme abgeführt werden. Der Reaktor lässt sich dadurch auch adiabat betreiben, wobei der Temperaturanstieg der Reaktionslösung durch den gekühlten Kreislaufstrom begrenzt werden kann. Da der Reaktor selbst dann nicht gekühlt werden muss, ist eine einfache und kostengünstige Bauform möglich. Eine Alternative stellt ein gekühlter Rohrbündelreaktor (nur im Fall des Festbetts) dar. Auch eine Kombination der beiden Fahrweisen ist denkbar. Hierbei wird bevorzugt ein Festbett- einem Suspensionsreaktor nachgeschaltet.

Das erfindungsgemäße Verfahren liefert als Hauptprodukt das lineare Ethylenamin (C₆-Produkt) TETA (1. Fall) sowie weitere Ethylenamine als Nebenkomponenten. Sofern im erfindungsgemäßen Verfahren ein Aminonitrilgemisch enthaltend EDDN und EDMN eingesetzt wird, erhält man ein Ethylenamingemisch, das als Hauptkomponente die beiden linearen Ethylenamine (C₆-Produkt und C₄-Produkt) TETA und DETA (2. Fall) sowie als Nebenkomponenten weitere Ethylenamine enthält.

Die Nebenkomponenten können in beiden Fällen sowohl lineare als auch cyclische Ethylenamine oder sonstige Nebenprodukte sein. Als wichtigstes cyclisches Nebenprodukt wird im 1. Fall AEPip (C₆-(Neben)Produkt) gebildet. Das Verhältnis von TETA zu AEPip im Produkt liegt normalerweise zwischen 3:1 bis 20:1.Dieses Verhältnis kann beispielsweise durch die Wahl des Lösungsmittels, des Katalysators, die Menge an Wasser und/oder die Zugabe eines Additivs gesteuert werden. Im 1. Fall ist DETA ebenfalls ein (lineares) Nebenprodukt. Als weitere Nebenreaktionen gibt es Zersetzungsreaktionen, die aber insbesondere durch die Wahl des Lösungsmittels, der Zudosiergeschwindigkeit, der Eduktreinheit und/oder Katalysators gesteuert und minimiert werden können. Im 2. Fall tritt Pip als weiteres wichtiges cyclisches Nebenprodukt (C₄-(Neben)Produkt) auf, das hauptsächlich aus EDMN gebildet ist. Bezüglich der Bildung und Steuerung des Verhältnisses DETA zu Pip gelten die gleichen Aussagen wie bei TETA zu AEPip. Das erfindungsgemäße Verfahren wird im nachfolgenden Schema 1 anhand des 2. Falls verdeutlicht, bei dem EDDN und EDMN gemeinsam beispielhaft ausgehend von FACH hergestellt werden.

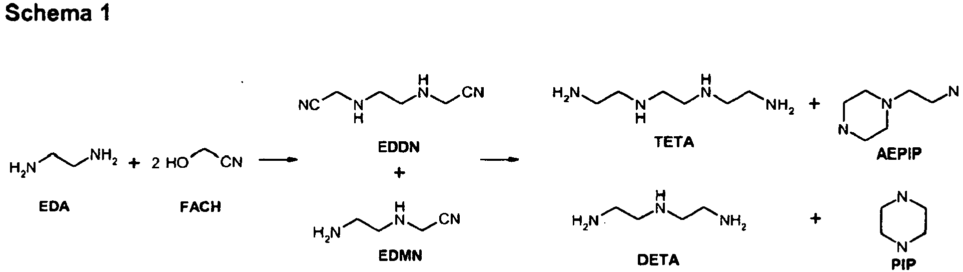

Im zweiten Fall wird der Begriff "Ethylenamingemisch" deswegen verwendet, weil das Reaktionsprodukt zwei lineare Ethylenamine als Hauptkomponenten (TETA und DETA) enthält, während im ersten Fall nur ein lineares Ethylenamin als Hauptprodukt (TETA) vorliegt. Die vorstehend bzw. nachfolgend aufgeführten Nebenprodukte werden folglich für die Begriffsdefinition in diesen beiden Fällen nicht berücksichtigt.

Im ersten Fall erhält man TETA mit einer Selektivität von vorzugsweise ≥ 70 Gew.-%, insbesondere ≥ 85 Gew.-%, besonders bevorzugt > 90% bezogen auf die eingesetzte Menge an EDDN. Im zweiten Fall findet sich das Verhältnis der Edukte EDDN und EDMN prinzipiell nach der Hydrierung bei den entsprechenden Produkten TETA und DETA wieder.

Unter dem Begriff "weiteres Ethylenamin" soll im Rahmen der vorliegenden Erfindung jede von TETA (1. Fall) und von TETA und DETA (2. Fall) verschiedene kohlenwasserstoffhaltige Verbindung verstanden werden, die mindestens zwei Ethyleneinheiten und mindestens zwei funktionelle Gruppen enthält, wobei die funktionellen Gruppen ausgewählt sind aus einer primären, einer sekundären oder einer tertiären Aminogruppe. Als weiteres Ethylenamin sollen im Rahmen der vorliegenden Erfindung auch cyclische Verbindungen wie beispielsweise Piperazin (Pip) sowie dessen Derivate verstanden werden. Ebenfalls soll Ethylendiamin (EDA) als weiteres Ethylenamin aufgefasst werden. Weitere Ethylenamine sind insbesondere Diethylentriamin (DETA; nur 1. Fall), Piperazin (Pip), Aminoethylenpiperazin (AEPip) oder Tetraethylenpentamin (TEPA).

Im Anschluss an die Hydrierung kann das erhaltene Produkt (TETA oder Ethylenamingemisch) gegebenenfalls weiter aufgereinigt werden, beispielsweise indem das Lösungsmittel und/oder der Katalysator nach dem Fachmann bekannten Methoden abgetrennt werden. Insbesondere können die Hauptprodukte (TETA und gegebenenfalls DETA) gemeinsam oder einzeln nach dem Fachmann bekannten Methoden aus dem Reaktionsprodukt isoliert werden. Sofern die beiden Hauptprodukte gemeinsam isoliert werden, beispielsweise durch eine Destillation, können sie anschließend in die beiden Einzelprodukte isoliert werden. Letztendlich erhält man somit reines TETA und reines DETA. Sonstige Verunreinigungen, Nebenprodukte oder weitere Ethylenamine wie TEPA oder Pip können ebenfalls mit dem Fachmann bekannten Methoden aus dem jeweiligen Produkt abgetrennt werden. Gegebenenfalls kann TETA auch gemeinsam mit in geringen Mengen gebildeten Diaminoethylpiperazin oder Piperazinyl-ethylethylendiamin isoliert werden.

In einer bevorzugten Ausführungsform wird das erfindungsgemäße Verfahren unter Verwendung von Tetrahydrofuran oder Methanol als Lösungsmittel durchgeführt. Die Temperatur bei der Hydrierung beträgt vorzugsweise 80 bis 140°C, der Druck vorzugsweise 40 bis 120 bar. Vorzugsweise wird die Hydrierung in Anwesenheit von EDA und/oder gegebenenfalls von Ammoniak durchgeführt.

Die nachfolgenden Beispiele verdeutlichen das erfindungsgemäße Verfahren. Die Anteile sind in Gew.-% angegeben, sofern nicht anders aufgeführt. Ein mitgeführter interner Standard, Diethylenglykoldimethylether (DEGDME), erlaubt eine Quantifizierung des Produktes durch Bestimmung der eventuell gebildeten flüchtigen Zersetzungsbestandteile. Die Quantifizierung erfolgt mittels Gaschromatographie (GC), wobei den jeweils entnommenen Proben zur Homogenisierung Methanol zugegeben wird.

### Beispiels:

### Allgemeine Vorschrift zur Synthese von Formaldehydcyanhydrin (FACH)

### Variante a)

In einem 61-Reaktionsgefäß mit Propellerrührer werden 6000 g (60 mol) Formaldehyd (30 %) vorgelegt und mit Natronlauge (1 mol/l) ein pH-Wert von 5.5 eingestellt. Innerhalb 2.5 Stunden werden 1661 g (61.2 mol) Blausäure über ein beheiztes U-Rohr unterhalb des Rührers gasförmig eindosiert, wobei die Reaktionstemperatur bei 30°C und der pH-Wert bei 5.5 gehalten wird. Nach 30 Minuten Nachrührzeit wird der pH-Wert mit Schwefelsäure (50 %ig) auf 2.5 gestellt. Über Liebig-Titration wird der entsprechende Gehalt ermittelt.

### Variante b)

In einem 61-Reaktionsgefäß mit Propellerrührer werden 7000 g (70 mol) Formaldehyd (30 %) vorgelegt und mit Natronlauge (1 mol/l) ein pH-Wert von 5.5 eingestellt. Innerhalb 3 Stunden werden 1938 g (71,4 mol) Blausäure über ein auf 50 °C beheiztes U-Rohr unterhalb des Rührers gasförmig eindosiert, wobei die Reaktionstemperatur bei 30 °C und der pH-Wert bei 5.5 gehalten wird. Nach 10 Minuten Nachrührzeit wird der pH-Wert mit Schwefelsäure (50 %ig) auf 2.5 gestellt. Um Leichtsieder, insbesondere Blausäure abzutrennen, wird der Reaktionsaustrag einer Sambaydestillation (wie in "Chemie Ingenieur Technik, Vol. 27, S. 257-261 beschrieben) (1 mbar, 30 °C) unterzogen. Über Liebig-Titration wird der entsprechende Gehalt ermittelt und gegebenenfalls durch Zugabe von Wasser ein Gehalt von 43-44 % bzw. 67 % FACH eingestellt.

### Beispiel 1:

### Formaldehydcyanhydrin

FACH wird nach der allgemeinen Vorschrift nach Variante a) hergestellt.

### Ethylendiamindiacetonitril

In einem 21-Reaktionsgefäß werden 536.5 g (4 mol) FACH (42.5 %) vorgelegt und unter Eiskühlung bei einer Temperatur von maximal 35 °C innerhalb von 2 Stunden 132 g (2.2 mol) Ethylendiamin zugetropft. Der Reaktionsansatz verfärbt sich von leicht gelb über orange nach braun. Nach kurzer Nachrührzeit wird die freie Blausäure durch Strippen mit Stickstoff entfernt (Volhard-Titration). Es wird laut Liebig-Titration ein Umsatz an FACH von 97.2 % erhalten.

### Triethylentetramin

a) Die erhaltene Ware wird im Semi-Batch-Verfahren hydriert. Hierbei werden in einem 270mL-Autoklaven 3,25g eines Cr-dotierten Raney-Cobalt Katalysators sowie 15ml THF vorgelegt. Der Autoklav wird auf 120°C hochgeheizt und Wasserstoff bis zu einem Gesamtdruck von 100bar aufgepresst. Innerhalb von 120 Minuten wird eine Mischung aus 13,8g der Roh-EDDN-Lösung, 13,8g eines internen Standards sowie 4,2g Wasser in 106g THF zudosiert. Die Reaktionsmischung wird weitere 60 Minuten unter Reaktionsbedingungen gerührt. Der Austrag wird mittels Methanol homogenisiert. Die Selektivität liegt bei 28% AEPip sowie 30% TETA. Desweiteren werden 4 Gew% an C4-Produkten (Pip + DETA) gefunden.
b) Die gleiche Ware wird ebenfalls im Semi-Batch-Verfahren hydriert. Hierbei werden in einem 270mL-Autoklaven 3,25g eines Cr-dotierten Raney-Cobalt Katalysators sowie 15ml THF und 5,4g EDA vorgelegt. Der Autoklav wird auf 120°C hochgeheizt und Wasserstoff bis zu einem Gesamtdruck von 100bar aufgepresst. Innerhalb von 120 Minuten wird eine Mischung aus 13,8g der Roh-EDDN-Lösung, 13,8g eines internen Standards sowie 4,2g Wasser in 106g THF zudosiert. Die Reaktionsmischung wird weitere 60 Minuten unter Reaktionsbedingungen gerührt. Der Austrag wird mittels Methanol homogenisiert. Die Selektivität liegt bei 12% AEPip sowie 43% TETA. Desweiteren wurden 4 Gew% an C4-Produkten (Pip + DETA) gefunden.
   Im Vergleich zu Beispiel 1a zeigt sich ein positiver Einfluss von EDA auf die TETA-Bildung.

### Beispiel 2:

### Formaldehydcyanhydrin

FACH wird nach der allgemeinen Vorschrift nach Variante b) hergestellt.

### Ethylendiamindiacetonitril

In einem 21-Reaktionsgefäß werden 132 g (2.2 mol) EDA vorgelegt und unter Eiskühlung bei einer Temperatur von maximal 30 °C innerhalb von 2 Stunden 511.2 g (4 mol) FACH (44.6 %) zugetropft. Nach 4.5 Stunden Nachrührzeit wird die leicht gelbe Lösung abgefüllt. Der Umsatz FACH beträgt laut Liebig Titration 99.2 %. Der Reaktionsansatz enthält 0.11 % freie Blausäure (bestimmt durch Volhard-Titration). Durch Titration wird eine EDDN-Ausbeute von 91,7 % bezogen auf eingesetztes FACH erhalten. EDMN lässt sich durch Titration nicht bestimmen. Unter der Annahme, dass aus umgesetztem Ethylendiamin, welches nicht zu EDDN reagiert, EDMN gebildet wird, ergibt sich als Gesamt-Aminonitril-Ausbeute 95,7 % und somit eine Ausbeute an EDMN von 4 %.

### Triethylentetramin

a) erhaltene Ware wird im Semi-Batch-Verfahren hydriert. Hierbei werden in einem 270mL-Autoklaven 3,25g eines Cr-dotierten Raney-Cobalt Katalysators sowie 15ml THF vorgelegt. Der Autoklav wird auf 120°C hochgeheizt und Wasserstoff bis zu einem Gesamtdruck von 100bar aufgepresst. Innerhalb von 120 Minuten wird eine Mischung aus 13,8g der Roh-EDDN-Lösung, 13,8g eines internen Standards sowie 4,2g Wasser in 106g THF zudosiert. Die Reaktionsmischung wird weitere 60 Minuten unter Reaktionsbedingungen gerührt. Der Austrag wird mittels Methanol homogenisiert. Die Selektivität liegt bei 27% AEPip sowie 47% TETA. Desweiteren werden 8 Gew% an C4-Produkten nachgewiesen.
   Es zeigt sich, dass durch die Leichtsieder-Abtrennung nach der FACH-Synthese eine deutlich bessere Ausbeute an Ethylenaminen erzielt werden kann. Durch den Überschuss an EDA in der EDDN-Synthese wird EDMN gebildet, welches zu den C4-Produkten DETA und Pip hydriert wird.
b) Die gleiche Ware wird ebenfalls im Semi-Batch hydriert. Hierbei werden in einem 270mL-Autoklaven 3,25g eines Cr-dotierten Raney-Cobalt Katalysators sowie 15ml THF und 13,5g EDA vorgelegt. Der Autoklav wird auf 120°C hochgeheizt und Wasserstoff bis zu einem Gesamtdruck von 100bar aufgepresst. Innerhalb von 120 Minuten wird eine Mischung aus 13,8g der Roh-EDDN-Lösung, 13,8g eines internen Standards sowie 4,2g Wasser in 106g THF zudosiert. Die Reaktionsmischung wird weitere 60 Minuten unter Reaktionsbedingungen gerührt. Der Austrag wird mittels Methanol homogenisiert. Die Selektivität liegt bei 8% AEPip sowie 82% TETA. Desweiteren werden 16 Gew% an C4-Produkten nachgewiesen.
   Durch die EDA-Zugabe in Variante 2 b) wird mehr lineares TETA gebildet. Ebenfalls zeigt sich ein Anstieg an C4-Produkten, der auf EDA-Kondensation beruht. Bei der Gew.-%-Angabe der C4-Produkte ist die Gewichtszunahme durch EDA-Kondensation mitberücksichtigt.

### Beispiel 3:

### Formaldehydcyanhydrin

FACH wird nach der allgemeinen Vorschrift nach Variante b) hergestellt.

### Ethylendiamindiacetonitril

In einem 21-Reaktionsgefäß werden 132 g (2.2 mol) EDA vorgelegt und unter Eiskühlung bei einer Temperatur von maximal 30 °C innerhalb von etwa 2 Stunden 340,8 g (4 mol) FACH (67 %) zugetropft. Nach 3 Stunden Nachrührzeit wird die gelbe Lösung abgefüllt. Der Umsatz FACH beträgt laut Liebig Titration 99.5 %. Der Reaktionsansatz enthält 0.08 % freie Blausäure (bestimmt durch Volhard-Titration). Durch Titration wird eine EDDN-Ausbeute von 82,9 % bezogen auf eingesetztes FACH erhalten. EDMN lässt sich durch Titration nicht bestimmen. Unter der Annahme, dass aus umgesetztem Ethylendiamin, welches nicht zu EDDN reagiert, EDMN gebildet wird, ergibt sich als Gesamt-Aminonitril-Ausbeute 90,5 % und somit eine Ausbeute an EDMN von 8 %.

### Triethylentetramin

a) Die erhaltene Ware wird im Semi-Batch-Verfahren hydriert. Hierbei werden in einem 270mL-Autoklaven 3,25g eines Cr-dotierten Raney-Cobalt Katalysators sowie 15ml THF vorgelegt. Der Autoklav wird auf 120°C hochgeheizt und Wasserstoff bis zu einem Gesamtdruck von 100bar aufgepresst. Innerhalb von 120 Minuten wird eine Mischung aus 13,8g der Roh-EDDN-Lösung, 13,8g eines internen Standards sowie 10g Wasser in 106g THF zudosiert. Die Reaktionsmischung wird weitere 60 Minuten unter Reaktionsbedingungen gerührt. Der Austrag wird mittels Methanol homogenisiert. Die Selektivität liegt bei 10% AEPip sowie 69% TETA. Zusätzlich werden 13% an C4-Produkten (Pip und DETA) erhalten.
   Für die Vergleichbarkeit wird gegenüber zu Beispiel 2a mehr Wasser zugegeben. Durch den Überschuss an EDA in der EDDN-Synthese wird EDMN gebildet, welches zu den C4-Produkten DETA und Pip hydriert wird.
b) Die erhaltene Ware wird im Semi-Batch-Verfahren hydriert. Hierbei werden in einem 270mL-Autoklaven 3,25g eines Cr-dotierten Raney-Cobalt Katalysators, 15ml THF und 13,5g EDA vorgelegt. Der Autoklav wird auf 120°C hochgeheizt und Wasserstoff bis zu einem Gesamtdruck von 100bar aufgepresst. Innerhalb von 120 min wird eine Mischung aus 13,8g der Roh-EDDN-Lösung, 13,8g eines internen Standards sowie 10g Wasser in 106g THF zudosiert. Die Reaktionsmischung wird weitere 60 Minuten unter Reaktionsbedingungen gerührt. Der Austrag wird mittels Methanol homogenisiert. Die Selektivität liegt bei 5% AEPip sowie 76% TETA. Desweiteren werden 16% an C4-Produkten erhalten.
   Durch die EDA-Zugabe wird mehr lineares TETA gebildet. Ebenfalls zeigt sich ein Anstieg an C4-Produkten, was auf EDA-Kondensation beruht.
c) Die erhaltene Ware wird im Semi-Batch-Verfahren hydriert. Hierbei werden in einem 270mL-Autoklaven 3,25g eines Cr-dotierten Raney-Cobalt Katalysators sowie 15ml THF vorgelegt. Der Autoklav wird auf 120°C hochgeheizt und Wasserstoff bis zu einem Gesamtdruck von 100bar aufgepresst. Innerhalb von 120 Minuten wird eine Mischung aus 13,8g der Roh-EDDN-Lösung, 13,8g eines internen Standards in 106g THF zudosiert. Die Reaktionsmischung wird weitere 60 Minuten unter Reaktionsbedingungen gerührt. Der Austrag wird mittels Methanol homogenisiert. Die Selektivität liegt bei 9% AEPip sowie 76% TETA. Zusätzlich wurden 12% an C4-Produkten (Pip und DETA) erhalten.
   Im Vergleich zu Beispiel 3a wird auf eine zusätzliche Zugabe an Wasser verzichtet, was sich positiv auf TETA auswirkt.

### Beispiel 4:

### Formaldehydcynhydrin

FACH wird nach der allgemeinen Vorschrift nach Variante b) hergestellt.

### Ethylendiamindiacetonitril

In einem 21-Reaktionsgefäß werden 132 g (2.2 mol) EDA vorgelegt und unter Eiskühlung bei einer Temperatur von maximal 50 °C innerhalb von 35 Minuten 340,8 g (4 mol) FACH (67 %) zugetropft. Nach 1 Stunde Nachrührzeit wird die fast klare Lösung abgefüllt. Der Umsatz FACH beträgt laut Liebig Titration 99.2 %. Der Reaktionsansatz enthält 0.07 % freie Blausäure (bestimmt durch Volhard-Titration). Durch Titration wird eine EDDN-Ausbeute von 87,7 % bezogen auf eingesetztes FACH erhalten. EDMN lässt sich durch Titration nicht bestimmen. Unter der Annahme, dass aus umgesetztem Ethylendiamin, welches nicht zu EDDN reagiert, EDMN gebildet wird, ergibt sich als Gesamt-Aminonitril-Ausbeute 93 % und somit eine Ausbeute an EDMN von 5 %.

### Triethylentetramin

a) Die erhaltene Ware wird im Semi-Batch-Verfahren hydriert. Hierbei werden in einem 270mL-Autoklaven 3,25g eines Cr-dotierten Raney-Cobalt Katalysators sowie 15ml THF vorgelegt. Der Autoklav wird auf 120°C hochgeheizt und Wasserstoff bis zu einem Gesamtdruck von 100bar aufgepresst. Innerhalb von 120 Minuten wird eine Mischung aus 13,8g der Roh-EDDN-Lösung, 13,8g eines internen Standards in 106g THF zudosiert. Die Reaktionsmischung wird weitere 60 Minuten unter Reaktionsbedingungen gerührt. Der Austrag wird mittels Methanol homogenisiert. Die Selektivität liegt bei 10% AEPip sowie 76% TETA. Zusätzlich werden 11% an C4-Produkten (Pip und DETA) erhalten.
   Versuch 4a bestätigt die Ergebnisse von 3c. Auch hier liegt durch den Überschuss an EDA in der EDDN-Synthese die Ausbeute an C4-Produkten (DETA und Pip) bei ca. 11%.
b) Die erhaltene Ware wird im Semi-Batch-Verfahren hydriert. Hierbei werden in einem 270mL-Autoklaven 3,25g eines Cr-dotierten Raney-Cobalt Katalysators, 15ml THF und 13,5g EDA vorgelegt. Der Autoklav wird auf 120°C hochgeheizt und Wasserstoff bis zu einem Gesamtdruck von 100bar aufgepresst. Innerhalb von 120 min wird eine Mischung aus 13,8g der Roh-EDDN-Lösung, 13,8g eines internen Standards in 106g THF zudosiert. Die Reaktionsmischung wird weitere 60 Minuten unter Reaktionsbedingungen gerührt. Der Austrag wird mittels Methanol homogenisiert. Die Selektivität liegt bei 4% AEPip sowie 80% TETA. Desweiteren werden 15% an C4-Produkten erhalten.
   In Beispiel 4b wird bestätigt, dass durch die Hydrierung in Gegenwart von EDA und einer geringeren Wassermenge, die AEPip-Bildung deutlich unterdrückt werden kann. Der Gehalt an 15 Gew% an C4-Produkten ist üblich, bei dem vorliegenden EDA-Überschuss in der EDDN-Synthese sowie EDA in der Hydrierung.

### Beispiel 5:

### Formaldehydcyanhydrin

FACH wird nach der allgemeinen Vorschrift nach Variante b) hergestellt.

### Ethylendiamindiacetonitril

In einem 21-Reaktionsgefäß werden 180 g (3 mol) EDA vorgelegt und unter Eiskühlung bei einer Temperatur von maximal 50 °C innerhalb von etwa 1 Stunde 511.2 g (6 mol) FACH (67 %) zugetropft. Nach 1,5 Stunden Nachrührzeit wird die hellgelbe Lösung abgefüllt. Der Umsatz FACH beträgt laut Liebig Titration 99.2 %. Der Reaktionsansatz enthält 0.02 % freie Blausäure (bestimmt durch Volhard-Titration). Durch Titration wird eine EDDN-Ausbeute von 92,6 % bezogen auf eingesetztes FACH erhalten. EDMN lässt sich durch Titration nicht bestimmen. Unter der Annahme, dass aus umgesetztem Ethylendiamin, welches nicht zu EDDN reagiert, EDMN gebildet wird, ergibt sich als Gesamt-Aminonitril-Ausbeute 94,5 % und somit eine Ausbeute an EDMN von 2 %.

### Triethylentetramin

a) Die erhaltene Ware wird im Semi-Batch-Verfahren hydriert. Hierbei werden in einem 270mL-Autoklaven 3,25g eines Cr-dotierten Raney-Cobalt Katalysators sowie 15ml THF vorgelegt. Der Autoklav wird auf 120°C hochgeheizt und Wasserstoff bis zu einem Gesamtdruck von 100bar aufgepresst. Innerhalb von 120 Minuten wird eine Mischung aus 13,8g der Roh-EDDN-Lösung, 13,8g eines internen Standards in 106g THF zudosiert. Die Reaktionsmischung wird weitere 60 Minuten unter Reaktionsbedingungen gerührt. Der Austrag wird mittels Methanol homogenisiert. Die Selektivität liegt bei 10% AEPip sowie 77% TETA. Zusätzlich werden 3% an C4-Produkten (Pip und DETA) erhalten.
   Es zeigt sich, dass durch den Einsatz von halbmolaren Mengen an EDA bei der EDDN-Synthese der Gehalt an C4-Produkten nach der Hydrierung lediglich 3% beträgt.
b) Die erhaltene Ware wird im Semi-Batch-Verfahren hydriert. Hierbei werden in einem 270mL-Autoklaven 3,25g eines Cr-dotierten Raney-Cobalt Katalysators, 15ml THF und 13,5g EDA vorgelegt. Der Autoklav wird auf 120°C hochgeheizt und Wasserstoff bis zu einem Gesamtdruck von 100bar aufgepresst. Innerhalb von 120 min wird eine Mischung aus 13,8g der Roh-EDDN-Lösung, 13,8g eines internen Standards in 106g THF zudosiert. Die Reaktionsmischung wird weitere 60 Minuten unter Reaktionsbedingungen gerührt. Der Austrag wird mittels Methanol homogenisiert. Die Selektivität liegt bei 6% AEPip sowie 82% TETA. Desweiteren werden 7% an C4-Produkten erhalten.
   Auch hier liegt der Gehalt an C4-Produkten deutlich unter demjenigen von Beispiel 4b.

Die vorstehenden Beispiele zeigen, dass die Qualität des eingesetzten FACHs Einfluss auf die Reaktionsdauer und die Farbe des Produktes bei der EDDN-Herstellung hat. Bei der anschließenden Hydrierung wird zudem eine höhere Selektivität erzielt, sofem das FACH destillativ aufgereinigt wird. Die Zugabe eines Additivs wirkt sich weiterhin positiv auf die Selektivität hinsichtlich linearer Ethylenamine aus. Die Wassermenge zeigt ebenfalls Einfluss auf die Bildung an linearem TETA.

### Beispiel 6:

### Formaldehydcyanhydrin

FACH wird nach der allgemeinen Vorschrift nach Variante b) hergestellt.

### Ethylendiamindiacetonitril

In einem 21-Reaktionsgefäß werden 132 g (2.2 mol) EDA vorgelegt und unter Eiskühlung bei einer Temperatur von maximal 50 °C innerhalb von 35 Minuten 340,8 g (4 mol) FACH (67 %) zugetropft. Nach 1 Stunde Nachrührzeit wird die fast klare Lösung abgefüllt. Der Umsatz FACH beträgt laut Liebig Titration 99.2 %. Der Reaktionsansatz enthält 0.07 % freie Blausäure (bestimmt durch Volhard-Titration). Durch Titration wird eine EDDN-Ausbeute von 87,7 % bezogen auf eingesetztes FACH erhalten. EDMN lässt sich durch Titration nicht bestimmen. Unter der Annahme, dass aus umgesetztem Ethylendiamin, welches nicht zu EDDN reagiert, EDMN gebildet wird, ergibt sich als Gesamt-Aminonitril-Ausbeute 93 % und somit eine Ausbeute an EDMN von 5 %.

### Triethylentetraamin

Die anschließende Hydrierung der oben erhaltenen Lösung wird kontinuierlich in einem 270mL Autoklav mit Stromstörern und Scheibenrührer durchgeführt. Hierbei werden 22g Cr-dotierter Raney-Cobalt vorgelegt und kontinuierlich 20NL Wasserstoff zugefahren. Pro Stunde werden 4,5g der EDDN-Lösung zusammen mit 2g eines internen Standards, 4,9g EDAsowie 30g THF zugefahren. Die Hydrierung wird bei 120°C und 100bar durchgeführt. Über einem Zeitraum von 26h können im Durchschnitt 2,6 Gew.-% Pip, 19,5 Gew% DETA an C4-Produkten sowie 5,6 Gew% AEPip und 79,9 Gew% TETA an C6-Produkten isoliert werden. Bezüglich EDDN entspricht dies einer Ausbeute von 96% an C6-Produkten.

### Beispiel 7:

### Formaldehydcyanhydrin

FACH wird nach der allgemeinen Vorschrift nach Variante b) hergestellt.

### Ethylendiamindiacetonitril

In einem 21-Reaktionsgefäß werden 120 g (2 mol) EDA vorgelegt und unter Eiskühlung bei einer Temperatur von maximal 70 °C innerhalb von 30 Minuten 340,8 g (4 mol) FACH (67 %) zugetropft. Nach 1 Stunde Nachrührzeit wird die klare gelb-orange Lösung abgefüllt. Der Umsatz FACH beträgt laut Liebig Titration 99,3 %. Der Reaktionsansatz enthält 0.12 % freie Blausäure (bestimmt durch Volhard-Titration). Durch Titration wird eine EDDN-Ausbeute von 91,6 % bezogen auf eingesetztes FACH erhalten. EDMN lässt sich durch Titration nicht bestimmen. Unter der Annahme, dass aus umgesetztem Ethylendiamin, welches nicht zu EDDN reagiert, EDMN gebildet wird, ergibt sich als Gesamt-Aminonitril-Ausbeute 94,3 % und somit eine Ausbeute an EDMN von 3 %.

### Triethylentetraamin

Die anschließende Hydrierung der oben erhaltenen Lösung wird kontinuierlich in einem 270mL Autoklav mit Stromstörern und Scheibenrührer durchgeführt. Hierbei werden 22g Cr-dotierter Raney-Cobalt vorgelegt und kontinuierlich 20NL Wasserstoff zugefahren. Pro Stunde werden 4,5g der EDDN-Lösung zusammen mit 2g eines internen Standards, 4,9g EDA sowie 30g THF zugefahren. Die Hydrierung wird bei 120°C und 100bar durchgeführt. Über einem Zeitraum von 26h können im Durchschnitt 2,4 Gew% Pip, 13,2 Gew% DETA an C4-Produkten sowie 4,8 Gew% AEPip und 84,1 Gew% TE-TA an C6-Produkten isoliert werden. Bezüglich EDDN entspricht dies einer Ausbeute von 98% an C6-Produkten.

### Beispiel 8: Belastungseinfluss in der Hydrierung

In einer eigenen Versuchsreihe wurde der Einfluss der Belastung lediglich auf das Verhältnis TETA/AEPip betracht.

### Formaldehydcyanhydrin

FACH wird nach der allgemeinen Vorschrift nach Variante a) hergestellt.

### Ethylendiamindiacetonitril

In einem 21-Reaktionsgefäß werden 132 g (2,2 mol) EDA vorgelegt und unter Eiskühlung bei einer Temperatur von maximal 35 °C innerhalb von 1,5 Stunden 506,6 g (4 mol) FACH (45 %ig) zugetropft. Nach 1 Stunde Nachrührzeit werden 14,3 g (0,1 mol) FACH (45 %ig) nachdosiert und auf 40 °C erwärmt. Es wird laut Liebig-Titration ein Umsatz an FACH von etwa 100 % erhalten.

### (EDDN-Hydrierung verschiedene Belastungen)

In einem 270mL-Autoklaven wird 3,25g (trocken) eines Cr-dotierten Raney-Cobalt Katalysators sowie 15ml THF vorgelegt. Der Autoklav wird auf 120°C hochgeheizt und Wasserstoff bis zu einem Gesamtdruck von 200bar aufgepresst. Innerhalb von einem definierten Zeitraum wird 13,8g der oben erhaltenen wässrigen EDDN-Lösung, 13,8g eines internen Standards sowie 4,2g Wasser in 106g THF zudosiert. Die Reaktionsmischung wird weitere 60 Minuten unter Reaktionsbedingungen gerührt. Zu unterschiedlichen Zeiträumen werden Proben genommen, die mittels Methanol homogenisiert werden. Nach vollständiger Zugabe kann kein EDDN nachgewiesen werden.

Das Verhältnis an TETA/AEPip wird bestimmt gemäß
a) 60min Zugabe: TETA/AEPip: 2,2
b) 180min Zugabe: TETA/AEPip: 3,3
c) 180min Zugabe: TETA/AEPip: 4,5

Bei 80°C Hydriertemperatur und 60min Zudosierung kann lediglich ein TETA/AE-Pip-Verhältnis von 1,3 erzielt werden.

### Beispiel 9 (Ammoniak als Additiv):

Für die Hydrierung in Gegenwart von Ammoniak wird die in Beispiel 7 erhaltene EDDN-Lösung eingesetzt.
a) In einem 270mL-Autoklaven werden 3,25g (trocken) eines Cr-dotierten Raney-Cobalt Katalysators, 15ml THF und 5,2g EDA vorgelegt. Der Autoklav wird auf 120°C hochgeheizt und Wasserstoff bis zu einem Gesamtdruck von 200bar aufgepresst. Innerhalb von 60 Minuten werden 13,8g der oben erhaltenen wässrigen EDDN-Lösung (43 Gew.-%), 13,8g eines internen Standards sowie 4,2g Wasser in 106g THF zudosiert. Die Reaktionsmischung wird weitere 60 Minuten unter Reaktionsbedingungen gerührt. Zu unterschiedlichen Zeiträumen werden Proben genommen, die mittels Methanol homogenisiert werden. Nach vollständiger Zugabe kann kein EDDN nachgewiesen werden. Nach 60minütiger Nachhydrierzeit liegt das Verhältnis an TETA und AEPip bei 4,1.
   In einem weiteren Versuch wird zusätzlich zum EDA 12g Ammoniak vorgelegt. Hierdurch konnte das Verhältnis auf 9,0 erhöht werden.
b) In einem 270mL-Autoklaven werden 3,25g (trocken) eines Cr-dotierten Raney-Cobalt Katalysators, 15ml THF und 12g Ammoniak vorgelegt. Der Autoklav wird auf 120°C hochgeheizt und Wasserstoff bis zu einem Gesamtdruck von 200bar aufgepresst. Innerhalb von 60 Minuten werden 13,8g der oben erhaltenen wässrigen EDDN-Lösung (43 Gew.-%), 13,8g eines internen Standards sowie 4,2g Wasser in 106g THF zudosiert. Die Reaktionsmischung wird weitere 60 Minuten unter Reaktionsbedingungen gerührt. Zu unterschiedlichen Zeiträumen werden Proben genommen, die mittels Methanol homogenisiert werden. Nach vollständiger Zugabe kann kein EDDN nachgewiesen werden. Nach 60minütiger Nachhydrierzeit liegt das Verhältnis an TETA und AEPip bei 5,7.

## Patentansprüche

1. Verfahren zur Herstellung von Triethylentetraamin (TETA), wobei Ethylendiamindiacetonitril (EDDN) in Gegenwart eines Katalysators und eines Lösungsmittels hydriert wird und der Druck 30 bis 250 bar beträgt.

2. Verfahren gemäß Anspruch 1, wobei EDDN in einem Aminonitrilgemisch enthaltend mindestens 30 Gew.-% EDDN und mindestens 5 Gew.-% Ethylendiaminmonoacetonitril (EDMN) vorliegt

3. Verfahren gemäß Anspruch 1 oder 2, **dadurch gekennzeichnet, dass** ein Raney-Katalysator eingesetzt wird, bevorzugt ein Raney-Nickel- oder ein Raney-Kobalt-Katalysator, insbesondere ein Raney-Kobalt-Skelett-Katalysator, der aus einer Co/Al-Legierung durch Laugung mit wässriger Alkalimetallhydroxid-Lösung erhalten wurde und der als Promotor mindestens eines der Elemente Fe, Ni oder Cr enthält.

4. Verfahren gemäß einer der Ansprüche 1 bis 3, **dadurch gekennzeichnet, dass** das Lösungsmittel Wasser und/oder ein organisches Lösungsmittel, insbesondere Tetrahydrofuran oder Methanol, ist.

5. Verfahren gemäß einem der Ansprüche 1 bis 4, **dadurch gekennzeichnet, dass** der Druck 40 bis 160 bar und/oder die Temperatur 70°C bis 140°C beträgt.

6. Verfahren gemäß einem der Ansprüche 1 bis 5, **dadurch gekennzeichnet, dass** im Aminonitrilgemisch 10 bis 25 Gew.-% EDMN enthalten ist.

7. Verfahren gemäß einem der Ansprüche 1 bis 6, **dadurch gekennzeichnet, dass** nach der Hydrierung TETA und/oder Diethylentriamin (DETA) und gegebenenfalls weitere Ethylenamine, die als Nebenprodukte im jeweils erhaltenen Reaktionsprodukt enthalten sind, isoliert werden.

8. Verfahren gemäß einem der Ansprüche 1 bis 7, **dadurch gekennzeichnet, dass** EDDN und/oder EDMN durch Umsetzung von Ethylendiamin (EDA) und Formaldehydcyanhydrin (FACH) oder durch Umsetzung von EDA mit Formaldehyd und Blausäure hergestellt werden.

9. Verfahren gemäß einem der Ansprüche 1 bis 8, **dadurch gekennzeichnet, dass** das EDDN mit einer Rate der Hydrierung zugeführt wird, die nicht größer ist als die Rate, mit der EDDN mit Wasserstoff bei der Hydrierung reagiert.

10. Verfahren gemäß einem der Ansprüche 1 bis 9, **dadurch gekennzeichnet, dass** die Hydrierung in Gegenwart eines Additivs, insbesondere in Gegenwart von EDA oder Ammoniak, durchgeführt wird.

11. Verfahren gemäß einem der Ansprüche 1 bis 10, **dadurch gekennzeichnet, dass** EDDN eingesetzt wird, das weitgehend frei von Leichtsiedern ist.

## Claims

1. A process for preparing triethylenetetramine (TETA), which comprises hydrogenating ethylenediaminediacetonitrile (EDDN) in the presence of a catalyst and a solvent at a pressure of from 30 to 250 bar.

2. The process according to claim 1, wherein EDDN is present in an amino nitrile mixture comprising at least 30% by weight of EDDN and at least 5% by weight of ethylenediaminemonoacetonitrile (EDMN).

3. The process according to claim 1 or 2, wherein a Raney catalyst, preferably a Raney nickel or Raney cobalt catalyst, in particular a skeletal Raney cobalt catalyst which has been obtained from a Co/Al alloy by leaching with aqueous alkali metal hydroxide solution and comprises at least one of the elements Fe, Ni or Cr as promoter, is used.

4. The process according to any of claims 1 to 3, wherein the solvent is water and/or an organic solvent, in particular tetrahydrofuran or methanol.

5. The process according to any of claims 1 to 4, wherein the pressure is from 40 to 160 bar and/or the temperature is from 70°C to 140°C.

6. The process according to any of claims 1 to 5, wherein the amino nitrile mixture comprises from 10 to 25% by weight of EDMN.

7. The process according to any of claims 1 to 6, wherein TETA and/or diethylenetriamine (DETA) and optionally further ethylene amines which are comprised as by-products in the reaction product obtained in each case are isolated after the hydrogenation.

8. The process according to any of claims 1 to 7, wherein EDDN and/or EDMN is/are prepared by reaction of ethylenediamine (EDA) and formaldehyde cyanohydrin (FACH) or by reaction of EDA with formaldehyde and hydrocyanic acid.

9. The process according to any of claims 1 to 8, wherein the EDDN is fed to the hydrogenation at a rate which is not greater than the rate at which EDDN reacts with hydrogen in the hydrogenation.

10. The process according to any of claims 1 to 9, wherein the hydrogenation is carried out in the presence of an additive, in particular in the presence of EDA or ammonia.

11. The process according to any of claims 1 to 10, wherein EDDN which is largely free of low boilers is used.

## Revendications

1. Procédé pour la préparation de triéthylènetétraamine (TETA), de l'éthylènediaminodiacétonitrile (EDDN) étant hydrogéné en présence d'un catalyseur et d'un solvant et la pression valant 30 à 250 bars.

2. Procédé selon la revendication 1, l'EDDN se trouvant dans un mélange d'aminonitriles contenant au moins 30% en poids d'EDDN et au moins 5% en poids d'éthylènediaminomonoacétonitrile (EDMN).

3. Procédé selon la revendication 1 ou 2, **caractérisé en ce qu'**on utilise un catalyseur de Raney, de préférence un catalyseur à base de nickel ou de cobalt de Raney, en particulier un catalyseur à base d'un squelette de cobalt de Raney, qui a été obtenu à partir d'un alliage Co/Al par lessivage avec une solution aqueuse d'hydroxyde de métal alcalin et qui contient comme promoteur au moins un des éléments Fe, Ni ou Cr.

4. Procédé selon l'une quelconque des revendications 1 à 3, **caractérisé en ce que** le solvant est l'eau et/ou un solvant organique, en particulier le tétrahydrofuranne ou le méthanol.

5. Procédé selon l'une quelconque des revendications 1 à 4, **caractérisé en ce que** la pression est de 40 à 160 bars et/ou la température est de 70°C à 140°C.

6. Procédé selon l'une quelconque des revendications 1 à 5, **caractérisé en ce que** 10 à 25% en poids d'EDMN sont contenus dans le mélange d'aminonitriles.

7. Procédé selon l'une quelconque des revendications 1 à 6, **caractérisé en ce qu'**après l'hydrogénation, de la TETA et/ou de la diéthylènetriamine (DETA) et le cas échéant d'autres éthylèneamines, qui sont contenues comme produits secondaires dans le produit de réaction à chaque fois obtenu, sont isolées.

8. Procédé selon l'une quelconque des revendications 1 à 7, **caractérisé en ce que** l'EDDN et/ou l'EDMN sont préparés par transformation d'éthylènediamine (EDA) et de formaldéhydecyanhydrine (FACH) ou par transformation d'EDA avec du formaldéhyde et de l'acide cyanhydrique.

9. Procédé selon l'une quelconque des revendications 1 à 8, **caractérisé en ce que** l'EDDN est ajouté à l'hydrogénation à une vitesse qui n'est pas supérieure à la vitesse à laquelle l'EDDN réagit avec l'hydrogène lors de l'hydrogénation.

10. Procédé selon l'une quelconque des revendications 1 à 9, **caractérisé en ce que** l'hydrogénation est réalisée en présence d'un additif, en particulier en présence d'EDA ou d'ammoniac.

11. Procédé selon l'une quelconque des revendications 1 à 10, **caractérisé en ce qu'**on utilise de l'EDDN qui est dans une large mesure exempt de fractions à bas point d'ébullition.
